# EUROPEAN PATENT APPLICATION

(11) **EP 3 263 552 A1**
(43) Date of publication of application: **03.01.2018**
(21) Application number: 17020257.6
(22) Date of filing: 19.06.2017
(51) Int. Cl.: C07C 305/12, A61K 31/567, A61K 31/575

(54) **METHODS FOR PREPARING INTERMEDIATES FOR THE SYNTHESIS OF OBETICHOLIC ACID**

(30) Priority: 28.06.2016 CZ 20160385
(71) Applicant: Zentiva K.S., 102 37 Praha 10 (CZ)
(72) Inventor: Kubelka, Tomas, 142 00 Praha 4 (CZ); Slavikova, Marketa, 252 66 Libcice nad Vltavou (CZ); Zezula, Josef, 588 13 Polna (CZ); Cerny, Josef, 274 01 Slany (CZ)
(74) Representative: Jirotkova, Ivana

(57) **Abstract**

The present invention discloses a method for preparing the key intermediates of formula 2 (disilylated derivative) and enones of formula 3 (mixture of E/Z isomers) for synthesis of the active pharmaceutical ingredient - obeticholic acid of formula 1. The principle of the method is that it comprises a reaction of the compound of formula **8**, wherein R₁ is a C1 to C4 linear or branched alkyl, with trimethylsilyl iodide in the presence of a base and a mixture of a polar aprotic and non-polar solvent under an inert atmosphere at a temperature in the range of from 40°C to 70°C.

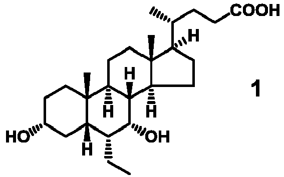

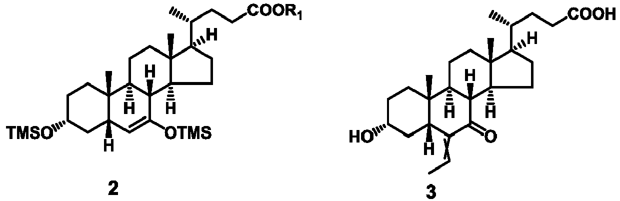

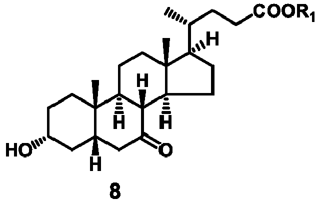

## Description

### Field of the Invention

The invention relates to a novel preparation method of the key intermediates for the synthesis of an active pharmaceutical ingredient - obeticholic acid of formula **1** (hereinafter only OCA, R. Pellicciari et al, J. Med. Chem. 2002, 45, 3569). OCA is a highly efficient and selective agonist of the farnesoid X receptor and is being developed for the treatment of primary biliary cirrhosis and non-alcoholic steatohepatitis.

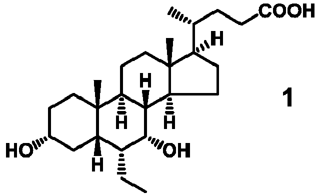

In particular, the invention relates to a new preparation method of the key intermediates, namely the disilylated derivative of formula **2** (wherein R₁ is a C1 to C4 linear or branched alkyl) and the enones of formula **3** (mixture of E/Z isomers).

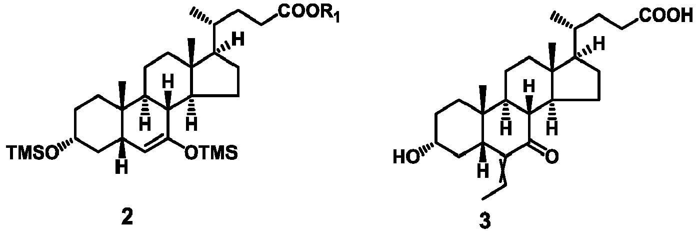

### Background Art

The application of the basic patent WO2002072598 describes a synthesis (Scheme 1) based on ketolithocholic acid **4,** which is converted to the tetrahydropyranyl derivative **5.** It is followed by enolization at extremely low temperatures (lower than -70°C) and alkylation by ethyl bromide in the presence of the toxic hexamethylphosphoramide (HMPA). The main drawbacks of this synthesis are low yields and the necessity to isolate the products by means of column chromatography.

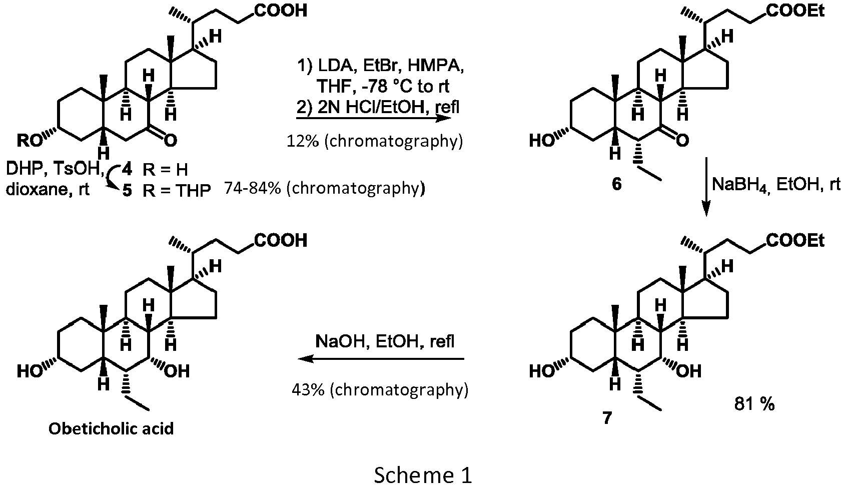

The first process patent application WO2006122977 prepares the disilylated intermediate **2** in two separate reactions and then converts it *in situ* to the enolate, which reacts with acetaldehyde. The product of this aldol reaction is then subject to dehydration and after saponification of the methyl ester the crystalline enone **3** (mainly the (*E*)-isomer) is isolated. Hydrogenation of the double bond provides the C6β ethyl ketone **9,** which is isomerized to the desired α isomer **10.** In the last step, the ketone is reduced to the respective alcohol **1** (Scheme 2).

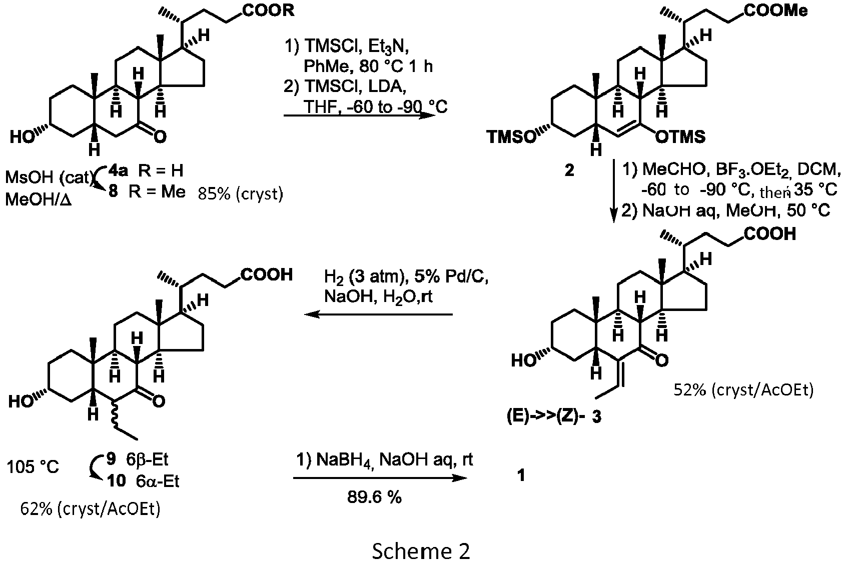

The subsequent patent application WO2013192097 represents a process improvement of the technology of the basic patent application WO2006122977.

The main improvement is unification of the two-step silylation and enolization into one step with the use of an excess of a strong base (lithium diisopropylamide - LDA) and chlorotrimethylsilane (TMSCI) at a low temperature of -25°C.

The rest of the synthesis follows the procedure of WO2006122977 with minor modifications of the type of changing the used solvent, changing the aqueous processing etc. with a positive impact on product purity.

As a precursor of the ethyl group acetaldehyde is used again, which has an extremely low boiling point at the atmospheric pressure (20°C), and therefore it must be used strongly subcooled. Therefore, its handling in a larger scale represents a certain risk.

### Disclosure of the Invention

Industrially usable preparation methods of the intermediates **2** and **3** have been developed. The preparation method of the bis(trimethylsilyl) derivative **2** uses trimethylsilyl iodide (TMSI), preferably generated *in situ,* which, in combination with a base at an advantageous reaction temperature (40 to 70°C), leads to the intermediate **2** in a high yield and purity.

The intermediate **2** is then used for a reaction with 1,1-dimethoxyethane, 1,1-diethoxyethane or 2,4,6-trimethyl-1,3,5-trioxane in the presence of a Lewis acid at elevated reaction temperatures (-70 to 25°C), providing the intermediate **3** in good yields and purity. The ratio of the *Z*>>*E* isomers of the enones **3** can be preferably reversed by means of isomerization under alkaline or acidic conditions. The intermediate **3** is preferably isolated in the form of a salt with amines.

The invention provides a method for preparing the intermediate of formula **2** 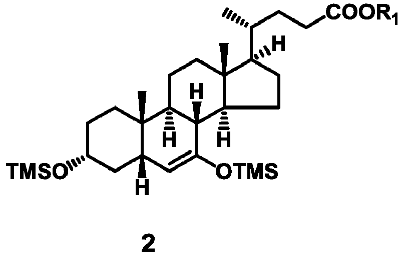 through a reaction of the compound of formula **8,** 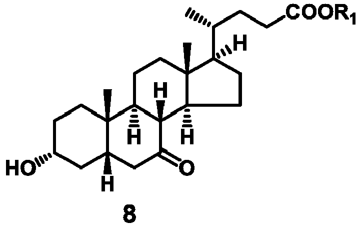 wherein R₁ is a C1 to C4 linear or branched alkyl, with trimethylsilyl iodide in the presence of a base and a mixture of a polar aprotic and non-polar solvent under an inert atmosphere at a temperature in the range from 40°C to 70°C. In some embodiments, trimethylsilyl iodide is generated *in situ* from a mixture of sodium iodide and trimethylsilyl chloride. In some embodiments, the base is selected from the group consisting of triethylamine, diisopropylethylamine and tri-*n*-butylamine. In some embodiments, the polar aprotic solvent is selected from the group consisting of acetonitrile, *N*,*N*-dimethylformamide, dichloromethane and 1,2-dichloroethane. In some embodiments, the non-polar solvent is selected from the group consisting of toluene, xylenes, heptane and hexane. In a preferred embodiment, the non-polar aprotic solvent is acetonitrile and the non-polar solvent toluene. In some embodiments, the reaction runs at a temperature in the range of 40 to 50°C.

The invention further provides a method for preparing a mixture of the enones of formula **3** 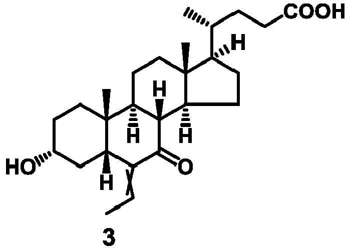 through a reaction of the compound of formula **2** 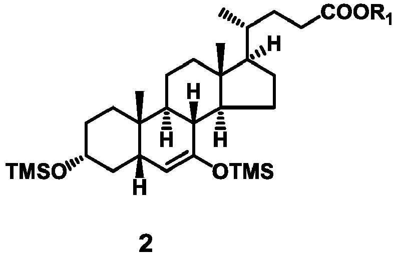 with an alkylating agent selected from the group consisting of 1,1-dimethoxyethane, 1,1-diethoxyethane and 2,4,6-trimethyl-1,3,5-trioxane, in the presence of a Lewis acid selected from the group consisting of BF₃*Et₂O, BF₃*CH₃CN and TiCl₄ in dichloromethane, the reaction being carried out at a temperature in the range from -70°C to 25°C, followed by processing with an aqueous solution of an acid or base. In a preferred embodiment, the alkylating agent is 1,1-dimethoxyethane and the Lewis acid BF₃*CH₃CN.

The invention further provides a method for isomerizing a mixture of the enones of formula **3** 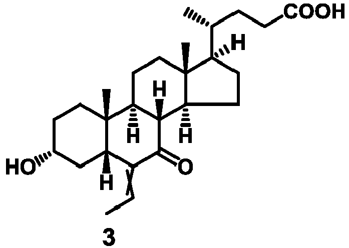 with a prevailing representation of the (*Z*)-isomer to a mixture with a prevailing representation of the (*E*)-isomer, comprising processing of the mixture by means of a strong acid or base in an alcoholic or aqueous-alcoholic solution at a temperature in the range from 0 to 60°C. In some embodiments, the acid is hydrochloric acid and the alcohol is methanol. In some embodiments, the base is sodium or potassium hydroxide and the aqueous-alcoholic solution is aqueous methanol. In some embodiments, the base is sodium ethoxide or methoxide in an alcoholic solution of ethanol or methanol.

The invention further provides a method for isolating and purifying the enone **3,** wherein the enone **3** is converted to a crystalline salt with an organic base. In some embodiments, the organic base is selected from the group consisting of *i*-isopropylamine, *t*-butylamine, cyclopentylamine and cyclohexylamine. The organic base is preferably *t*-butylamine.

The invention further provides a crystalline salt of the enone **3** with *t*-butylamine, *i-*isopropylamine, cyclopentylamine and/or cyclohexylamine.

### Detailed description of the Invention

The prior art of preparation of the intermediate **2** used either a two-step method of gradual silylation and enolization with the reaction temperature range from 80°C to -70°C (WO2006122977), or a single-step reaction with an excess of a strong base (LDA) and trimethylsilyl chloride at the low temperatures around -25°C (WO2013192097).

In the literature (Arendt, M. J. Prakt. Chem. 1998, 340, 760-763) a system for generation of highly reactive trimethylsilyl iodide through a reaction of sodium iodide with trimethylsilyl chloride in a mixture of toluene and acetonitrile at 50°C is described. This combination of agents has been successfully tested with steroidal substrates with a free carboxylic group (Königsberger, K. et al Org. Proc. Res. Dev. 2002, 6, 665-669) for the preparation of trimethylsilyl enol ethers.

According to well-known procedures various esters (e.g. methyl, ethyl) of ketolithocholic acid 4 were prepared and their reaction with trimethylsilyl iodide was tested in a mixture of a polar aprotic (e.g. acetonitrile, *N*,*N*-dimethylformamide, dichloromethane, 1,2-dichloroethane) and non-polar solvent (e.g. toluene, xylenes, heptane, hexane) at an elevated temperature (40 to 70°C) in the presence of a suitable base (e.g. triethylamine, diisopropylethylamine, tri-*n*-butylamine) under an inert atmosphere. Trimethylsilyl iodide was advantageously generated *in situ* by a mixture of the above mentioned agents. In our case, this combination of agents smoothly converted the esters of ketolithocholic acid **8** to the respective bisTMS (bis(trimethylsilyl)) derivatives **2,** surprisingly maintaining the ester group. An advantage of this method is its simplicity and low demands for cooling.

1,1-dimethoxyethane, 1,1-diethoxyethane and 2,4,6-trimethyl-1,3,5-trioxane were tested as the alkylation agents (Scheme 3) with the aim to increase the temperature of the subsequent reaction step and to avoid using the low-boiling and potentially toxic acetaldehyde to facilitate industrial production of the intermediate **3.** A reaction of the bisTMS derivatives **2** with the above mentioned agents in the presence of a suitable Lewis acid selected from the group consisting of boron fluoride diethyl etherate (BF₃*Et₂O), the boron fluoride/acetonitrile complex (BF₃*CH₃CN) and titanium chloride (TiCl₄) in dichloromethane at the temperatures in the range from -70°C to 25°C, followed by processing with an aqueous solution of an acid or base, provided the desired product - enone **3** as a mixture of the *E*/*Z* isomers.

It has been surprisingly found out that these agents made it possible to carry out the reaction with comparable results as with acetaldehyde, namely at much higher (-30 - 25°C), and therefore technologically more advantageous reaction temperatures.

It has further been surprisingly found out that in the product, the main isomer of the enone **3** at the double bond was the (Z)-isomer unlike the technology of the preceding patent applications WO2006122977 and WO2013192097, where the (*E*)-isomer prevails.

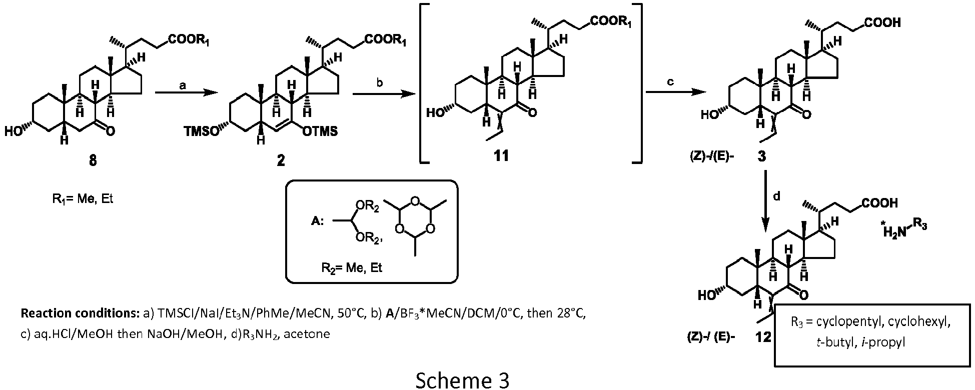

Stability of both the isomers of the enone **3** was also studied. When heated up in aqueous sodium hydroxide (conditions used in WO2006122977 and WO2013192097 during hydrogenation in the next step), both the isomers are subject to decomposition back to the input substance, the (*Z*)-isomer being more reactive than the (*E*)-isomer.

It has also been found out that the (*Z*)-isomer of the enone **3** could be advantageously isomerized either under controlled acidic (solutions of hydrochloric acid) or alkaline (solutions of sodium or potassium hydroxide, solutions of sodium ethoxide or methoxide) conditions to the better crystallizing (*E*)-isomer. Suitable solvents for the isomerizations are lower alcohols (methanol, ethanol), water and their mixtures at the reaction temperatures from 0 to 60°C.

Another substantial improvement involves finding crystalline salts of the enones **3** with primary amines as *i*-isopropylamine, *t*-butylamine, cyclopentylamine and cyclohexylamine.

These crystalline salts **12** make it possible to obtain the desired product **3** in a high purity and also in a higher yield than from the method with isolation of the free acid **3.** From the point of view of the yield and purity of the product, the salt of the acid **3** with *t*-butylamine is especially preferred.

### Examples

### Example 1

### Methyl (3α,5β)-3,7-bis[(trimethylsilyl)oxy]chol-6-en-24-oate of formula 2

The reaction was carried out in pre-dried apparatus under strictly anhydrous conditions in a nitrogen atmosphere. Sodium iodide (4.14 g; 27.6 mmol; 5.5 equiv.) was put in a flask and toluene (5 ml) and acetonitrile (5 ml) were added. TMSCI (3.18 ml; 25.1 mmol; 5 equiv.) was added to the resulting suspension under stirring during 10 min and the reaction mixture was stirred for one hour at the laboratory temperature (approx. 22°C). The methyl ester **8** (2.03 g; 5.02 mmol) was dissolved in toluene (5 ml) and acetonitrile (5 ml) and Et₃N (4.2 ml; 30.12 mmol; 6 equiv.) was added. The solution of the methyl ester **8** was pushed into the TMSI solution with a cannula and the reaction mixture was heated for 5 hours at the temperature of 50°C. Then, the reaction mixture was cooled down to 10°C and poured into a solution of citric acid monohydrate (2 g) in water (21 ml), cooled down to 10°C. The obtained mixture was stirred for 10 minutes. The aqueous phase was separated and removed. Water (10 ml) was added to the organic phase, the mixture was stirred for 10 minutes and the aqueous phase was separated and removed. The solvents were removed by distillation at 50-60°C and 300 hPa. The obtained intermediate **2** was used for the next step without further purification. The purity and identity of the intermediate **2** was verified by means of ¹H NMR spectroscopy.

### Example 2

### Methyl (3α,5β)-3,7-bis[(trimethylsilyl)oxy]chol-6-en-24-oate of formula 2

The reaction was carried out in pre-dried apparatus under strictly anhydrous conditions in a nitrogen atmosphere. The methyl ester **8** (150 g; 371 mmol) was put in a reactor with the content of 2l, toluene (350 ml) was added and the solution was heated up to the a temperature of 50-60°C and toluene (190 ml) was removed by distillation at a reduced pressure (300 hPa). Acetonitrile (150 ml), sodium iodide (150 g; 371 mmol) were added to the reaction mixture, Et₃N (62 ml; 444.9 mmol; 6 equiv.) was poured into the mixture and during 10 min TMSCI (47 ml; 370.8 mmol; 5 equiv.) was added dropwise. The reaction mixture was heated for 3.5 hours at 50°C. Then, the reaction mixture was cooled down to 10°C and released. A solution of citric acid monohydrate (15.44 g; 73.5 mmol; 0.99 equiv.) in water (300 ml) was poured into the reactor and cooled down to 10°C. The reaction mixture was poured into the cooled citric acid solution and the resulting mixture was stirred for 10 minutes. The aqueous phase was separated and removed. Water (80 ml) was added to the organic phase, the mixture was stirred for 10 minutes and the aqueous phase was separated and removed. The solvents were removed by distillation at 50-60°C and 300 hPa. The obtained intermediate **2** was used for the next step without further purification. The purity and identity of the intermediate **2** was verified by means of ¹H NMR spectroscopy.

### Example 3

### (3α,5β)-6-Ethylidene-3-hydroxy-7-oxocholan-24-oic acid of formula 3

The reaction was carried out in pre-dried apparatus under strictly anhydrous conditions in a nitrogen atmosphere. Dichloromethane (176 ml) was put in a reactor with the content of 1l and the solution was cooled down to the temperature of -30°C. BF₃.MeCN (145 ml; 185.35 mmol; 2.5 equiv.; 16% solution) was added to the solution. Then, at -30°C, during five minutes a solution of the intermediate **2** (40.7 g; 74.14 mmol; 1 equiv.) and 1,1-dimethoxyethane (23.5 ml; 222.4 mmol; 3 equiv.) in dichloromethane (50 ml) cooled down to -30°C was added. The reaction mixture was stirred for two hours at -30°C and during one hour it was heated up to the laboratory temperature and stirred for another two hours at the laboratory temperature. Then, the reaction mixture was cooled down to 10°C and released from the reactor. A solution of NaOH (7.57 g; 189.25 mmol; 2.55 equiv.) in water (170 ml) was put in the reactor and cooled down to 10°C. The reaction mixture was poured into a solution of NaOH and stirred for 10 minutes. Then, the aqueous phase was separated and removed and the organic phase was returned into the reactor. The organic solvents were evaporated at 55°C and 800 hPa. The evaporation product was cooled down to 20°C and suspended in MeOH (50 ml). A solution of NaOH (5.09 g; 127.25 mmol; 1.72 equiv.) in water (14 ml) was poured into the reaction mixture. The reaction mixture was heated up to 50°C and stirred at this temperature for three hours. Then, the reaction mixture was cooled down to 20°C, diluted with water (112 ml) and ethyl acetate (174 ml) was added. During five minutes. a solution of citric acid monohydrate (30.20 g; 144 mmol; 1.94 equiv.) in water (60 ml) was added to the reaction mixture under stirring. The reaction mixture was stirred for 10 minutes. The aqueous phase was separated and removed. The reaction mixture was concentrated by distillation at 50-60°C and 500 hPa. After cooling down to 20°C it was crystallized under stirring overnight. Crystals of the product were filtered and washed with ethyl acetate (50 ml) and dried at 50°C/180 hPa. The amount of 10.7 g (35%) of yellowish crystals was obtained, HPLC: **(*Z*)-3 -** 80.70%, **(*E*)-3 -** 15.34%.

### Example 4

### (3α,5β)-6-Ethylidene-3-hydroxy-7-oxocholan-24-oic acid of formula 3

The reaction was carried out in pre-dried apparatus under strictly anhydrous conditions in a nitrogen atmosphere. Dichloromethane (9.0 ml) was put in a flask and the solution was cooled down to the temperature of 0°C. BF₃*MeCN (7.58 ml; 9.53 mmol; 2.5 equiv.; 16% solution) was added to the solution. Then, at 0°C, during five minutes a solution of the intermediate **2** (2.09 g; 3.81 mmol; 1 equiv.) and 1,1-dimethoxyethane (1.21 ml; 11.42 mmol; 3 equiv.) in dichloromethane (2.5 ml) cooled down to 0°C was added. The reaction mixture was stirred for two hours at 0°C and during one hour it was heated up to the RT and stirred for another two hours at the RT. Then, the reaction mixture was cooled down to 10°C, poured into a solution of NaOH (252 mg) in water (8.38 ml) cooled down to 10°C and stirred for ten minutes. Then, the aqueous phase was separated and removed. The organic solvents were evaporated at 55°C and 800 mbar. The evaporation product of the reaction mixture was suspended in MeOH (4.2 ml). A solution of NaOH (362 mg) in water (1.1 ml) was poured into the reaction mixture. The reaction mixture was heated up to 50°C and stirred at this temperature for three hours. Then, the reaction mixture was cooled down to 20°C, diluted with water (10 ml) and ethyl acetate (10 ml) was added. 85% wt. phosphoric acid (0.5 ml) was added to the reaction mixture and the reaction mixture was stirred for 10 minutes. The aqueous phase was separated and removed. The reaction mixture was evaporated until dry. The amount of 0.8 g (50%) of yellowish-brown foam was obtained, HPLC: **(*Z*)-3 -** 52.77%, **(*E*)-3** - 13.47%.

### Example 5

### (3α,5β)-6-Ethylidene-3-hydroxy-7-oxocholan-24-oic acid of formula 3

The reaction was carried out in pre-dried apparatus under strictly anhydrous conditions in a nitrogen atmosphere. Dichloromethane (4.25 ml) was put in a flask and the solution was cooled down to the temperature of -60°C. BF₃.MeCN (3.5 ml; 4.55 mmol; 2.5 equiv.; 16% wt. solution) was added to the solution. Then, at -60°C, during five minutes a solution of the intermediate **2** (1.00 g; 1.82 mmol; 1 equiv.) and 2,4,6-trimethyl-1,3,5-trioxane (0.484 ml; 3.64 mmol; 2 equiv.) in dichloromethane (1.2 ml) cooled down to -60°C was added. The reaction mixture was stirred for two hours at -60°C and during one hour it was heated up to the laboratory temperature and stirred for another two hours at the laboratory temperature. Then, the reaction mixture was cooled down to 10°C, poured into a solution of NaOH (120 mg) in water (4.00 ml) cooled down to 10°C and stirred for ten minutes. Then, the aqueous phase was separated and removed. The organic solvents were evaporated at 55°C and 800 hPa. The evaporation product of the reaction mixture was suspended in MeOH (2.0 ml). A solution of NaOH (165 mg) in water (0.5 ml) was poured into the reaction mixture. The reaction mixture was heated up to 50°C and stirred at this temperature for three hours. Then, the reaction mixture was cooled down to 20°C, diluted with water (10 ml) and ethyl acetate (10 ml) was added. 85% wt. phosphoric acid (0.25 ml) was added to the reaction mixture and the reaction mixture was stirred for 10 minutes. The aqueous phase was separated and removed. The reaction mixture was evaporated until dry. The amount of 0.31 g (41%) of yellowish-brown foam was obtained, HPLC: **(*Z*)-3 -** 24.02%, **(*E*)-3 -** 43.67%.

### Example 6

### (3α,5β)-6-Ethylidene-3-hydroxy-7-oxocholan-24-oic acid of formula 3

The reaction was carried out in pre-dried apparatus under strictly anhydrous conditions in a nitrogen atmosphere. Dichloromethane (13.0 ml) was put in a flask and the solution was cooled down to the temperature of 0°C. BF₃*MeCN (10.69 ml; 13.66 mmol; 2.5 equiv.; 16% wt. solution) was added to the solution. Then, at 0°C, during five minutes a solution of the intermediate **2** (3.00 g; 5.47 mmol; 1 equiv.) and 1,1-dimethoxyethane (1.73 ml; 16.40 mmol; 3 equiv.) in dichloromethane (3.7 ml) cooled down to 0°C was added. The reaction mixture was stirred for two hours at 0°C and during one hour it was heated up to the laboratory temperature and stirred for another two hours at the laboratory temperature. Then, the reaction mixture was cooled down to 10°C, poured into a solution of NaOH (360 mg) in water (12 ml) cooled down to 10°C and stirred for ten minutes. Then, the aqueous phase was separated and removed. The organic solvents were evaporated at 55°C and 800 hPa. The evaporation product of the reaction mixture was suspended in MeOH (10 ml). 35% wt. HCl (2 ml) and water (1 ml) was poured into the reaction mixture. The reaction mixture was heated up to 50°C and stirred at this temperature for six hours. Then, the reaction mixture was cooled down to 20°C, diluted with water (10 ml) and ethyl acetate (10 ml) was added. The aqueous phase was removed. The reaction mixture was evaporated until dry. The evaporation product was suspended in methanol (6.0 ml). A solution of NaOH (520 mg) in water (1.6 ml) was poured into the reaction mixture. The reaction mixture was heated up to 50°C and stirred at this temperature for three hours. Then, the reaction mixture was cooled down to 20°C, diluted with water (20 ml) and ethyl acetate (10 ml) was added. 85% wt. phosphoric acid (0.5 ml) was added to the reaction mixture and the reaction mixture was stirred for 10 minutes. The aqueous phase was separated and removed. The reaction mixture was evaporated until dry. The amount of 2.28 g (75%) of yellowish-brown foam was obtained, HPLC: **(*Z*)-3 -** 20.86%, **(*E*)-3 -** 61.35%.

### Example 7

### (3α,5β)-6-Ethylidene-3-hydroxy-7-oxocholan-24-oic acid of formula 3

For the reaction, the intermediate **3** (0.1 g; 0.24 mmol) was used with the composition according to HPLC: **(*E*)-3**-22%, **(*Z*)-3**-71%. The input material was dissolved in methanol (1 ml) and hydrochloric acid (0.5 ml; 1M solution in water) was added. The reaction mixture was heated at 50°C for 19 hours. After cooling of the reaction mixture to 20°C, water (5 ml) was added and the product was extracted to ethyl acetate (5 ml). The organic phase was separated and evaporated until dry. An analysis of the obtained mixture by means of ¹H NMR determined the ratio **(*E*)-3**-72%, **(*Z*)-3**-18%.

### Example 8

### (3α,5β)-6-Ethylidene-3-hydroxy-7-oxocholan-24-oic acid of formula 3

The isolated enone **3** ((***E***)-:(***Z***)- 19 : 81; 0.017 g; 0.041 mmol) was suspended in 50% wt. aqueous sodium hydroxide (1 ml) and ethanol (1 drop) was added. The mixture was agitated at the laboratory temperature for 24 h. After adjustment to pH∼1 by addition of 35% wt. hydrochloric acid, the product was extracted to dichloromethane (10 ml total), the organic fraction was washed with water and evaporated. An analysis by means of ¹H NMR determined the ratio ((***E***)-:(***Z***)- 78 : 22), indicating efficient isomerization of the (***Z***)-enone to (***E***)-enone with only traces of ketolithocholic acid **4a.**

### Example 9

### Stability test of (3α,5β)-6-Ethylidene-3-hydroxy-7-oxocholan-24-oic acid

The isolated enone **3** ((***E***)-:(***Z***)- 19 : 81; 0.017 g; 0.041 mmol) was suspended in 50% wt. aqueous sodium hydroxide (1 ml) and ethanol (1 drop) was added. The mixture was stirred while being heated in an oil bath at 90°C for 24 h. After adjustment to pH∼1 by addition of 35% hydrochloric acid, the product was extracted to dichloromethane (10 ml total), the organic fraction was washed with water and evaporated. An analysis by means of ¹H NMR showed that the enones **3** got completely decomposed to ketolithocholic acid **4a.**

### Example 10

### t-Butyl ammonium salt of (3α,5β)-6-ethylidene-3-hydroxy-7-oxocholan-24-oic acid of formula 12

The reaction was carried out in pre-dried apparatus under strictly anhydrous conditions in a nitrogen atmosphere. Dichloromethane (2500 ml) was put in a reactor with the content of 251 and the solution was cooled down to the temperature of -30°C. BF₃.MeCN (2060 ml; 2.66 mmol; 2.5 equiv.; 16% solution) was added to the solution. Then, at -30°C, during five minutes, a solution of the intermediate **2** (583.4 g; 1.06 mol; 1 equiv.) and 1,1-dimethoxyethane (337 ml; 3.19 mol; 3 equiv.) in dichloromethane (720 ml) cooled down to -30°C was added. The reaction mixture was stirred for two hours at -30°C and then during one hour it was heated up to the temperature of 25°C and stirred for another two hours at the temperature of 25°C. Then, the reaction mixture was cooled down to 10°C and repumped to a pre-cooled (10°C) aqueous solution of NaOH (108.4 g; 2.71 mol; 2.55 equiv. of NaOH in water 2400 ml) in a reactor with the volume of 151. The obtained emulsion was stirred for 15 minutes, then the aqueous phase was separated and removed and the organic phase was returned into the reactor.

The organic solvents were evaporated at 55°C and 800 hPa. The evaporation product was suspended in MeOH (860 ml). A solution of hydrochloric acid (265 ml 36% HCl; 3.29 mol; 3 equiv.) in water (170 ml) was poured to the obtained solution. The obtained reaction mixture was heated up to 50°C and stirred for 1.5 h. Subsequently, the reaction mixture was cooled down to 40°C. A solution of NaOH (208 g; 5.21 mol; 4.9 equiv.) in water (465 ml) was poured into the reaction mixture. The reaction mixture was heated up to 50°C and stirred at this temperature for two hours. Then, the reaction mixture was cooled down to 20°C, diluted with water (1700 ml) and toluene (2500 ml). The layers were separated and isopropyl acetate (3000 ml) was added to the aqueous layer. During five minutes a solution of citric acid (430 g; 2.23 mol; 2.1 equiv.) in water (860 ml) was added to the reaction mixture under stirring.

The reaction mixture was stirred for 10 minutes. The aqueous phase was separated and removed. The reaction mixture was diluted with acetone (1300 ml) and heated up to the temperature of 40°C. During 10 minutes, *t*-butyl amine (134 ml; 1.28 mol; 1.2 equiv.) was added at the temperature of 40°C. The obtained suspension was cooled down to the temperature of 10°C and crystallized under stirring for 1 hour. Crystals of the product were filtered and washed with acetone (750 ml) and dried at 40°C and 180 hPa. The amount of 311 g (60%) of yellowish orange crystals of the ammonium salt **12** was obtained, HPLC: **(*E*)-3** - 79.92%, **(*Z*)-3** - 13.68%.

## Claims

1. A method for preparing the intermediate of formula **2,** 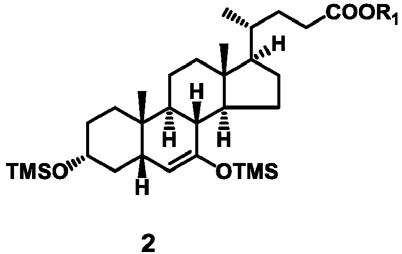 **characterized in that** it comprises a reaction of the compound of formula **8,** 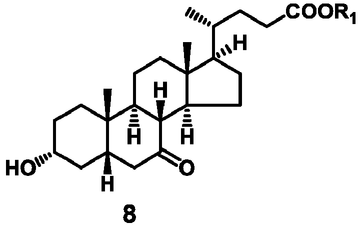 wherein R₁ is a C1 to C4 linear or branched alkyl, with trimethylsilyl iodide in the presence of a base and a mixture of a polar aprotic and non-polar solvent under an inert atmosphere at a temperature in the range of from 40°C to 70°C.

2. The method according to claim 1, **characterized in that** trimethylsilyl iodide is generated *in situ* from a mixture of sodium iodide and trimethylsilyl chloride.

3. The method according to claim 1, **characterized in that** the base is selected from the group consisting of triethylamine, diisopropylethylamine and tri-*n*-butylamine.

4. The method according to claim 1, **characterized in that** the polar aprotic solvent is selected from the group consisting of acetonitrile, *N*,*N*-dimethylformamide, dichloromethane and 1,2-dichloroethane.

5. The method according to claim 1, **characterized in that** the non-polar solvent is selected from the group consisting of toluene, xylenes, heptane and hexane.

6. The method according to claims 4 or 5, **characterized in that** the polar aprotic solvent is acetonitrile and the non-polar solvent is toluene.

7. The method according to claim 1, **characterized in that** the reaction runs at a temperature in the range of 40 to 50°C.

8. A method for preparing a mixture of the enones of formula **3,** 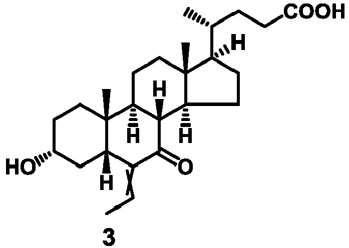 **characterized in that** it comprises a reaction of the compound of formula **2** 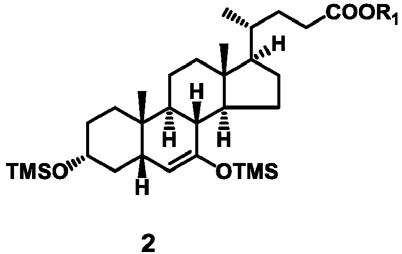 with an alkylating agent selected from the group consisting of 1,1-dimethoxyethane, 1,1-diethoxyethane and 2,4,6-trimethyl-1,3,5-trioxane, in the presence of a Lewis acid selected from the group consisting of BF₃*Et₂O, BF₃*CH₃CN and TiCl₄ in dichloromethane, the reaction being carried out at a temperature in the range of from -70°C to 25°C, followed by processing with an aqueous solution of an acid or base.

9. The method according to claim 8, **characterized in that** the alkylating agent is 1,1-dimethoxyethane and the Lewis acid is BF₃*CH₃CN.

10. A method for isomerizing a mixture of the enones of formula 3 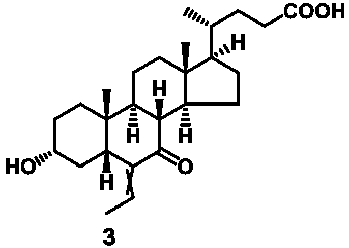 with a prevailing representation of the (*Z*)-isomer to a mixture with a prevailing representation of the (*E*)-isomer, **characterized in that** it comprises processing of the mixture by means of a strong acid or base in an alcoholic or aqueous-alcoholic solution at a temperature in the range of from 0 to 60°C.

11. The method according to claim 10, **characterized in that** the acid is hydrochloric acid and the alcohol is methanol.

12. The method according to claim 10, **characterized in that** the base is sodium or potassium hydroxide and the aqueous-alcoholic solution is aqueous methanol.

13. The method according to claim 10, **characterized in that** the base is sodium ethoxide or methoxide in an alcoholic solution of ethanol or methanol.

14. A method for isolating and purifying the enone 3, **characterized in that** the enone 3 is converted to a crystalline salt with an organic base.

15. The method according to claim 14, **characterized in that** the organic base is selected from the group consisting of i-isopropylamine, t-butylamine, cyclopentylamine and cyclohexylamine.

16. The method according to claim 15, **characterized in that** the organic base is t-butylamine.

17. A crystalline salt of the enone 3 with t-butylamine.
